# EUROPÄISCHE PATENTSCHRIFT

(11) **EP 3 200 875 B1**
(45) Veröffentlichungstag und Bekanntmachung des Hinweises auf die Patenterteilung: **27.06.2018**
(21) Anmeldenummer: 15810728.4
(22) Anmeldetag: 09.09.2015
(51) Int. Cl.: A61N 5/06, A61M 21/00

(54) **VORRICHTUNG ZUR SIGNALÜBERMITTLUNG ZUM AUGE**
APPARATUS FOR TRANSMITTING SIGNALS TO THE EYE
DISPOSITIF POUR LA TRANSMISSION D'UN SIGNAL À L'OEIL

(30) Priorität: 29.09.2014 AT 506942014
(43) Veröffentlichungstag der Anmeldung: 09.08.2017
(73) Patentinhaber: Pocket Sky OG, 1020 Wien (AT)
(72) Erfinder: GEYER, Michael, 1120 Wien (AT); WALLERBERGER, Mark, 1030 Wien (AT)
(74) Vertreter: KLIMENT & HENHAPEL
(86) Internationale Anmeldenummer: PCT/AT2015/050224
(87) Internationale Veröffentlichungsnummer: WO 2016/049669

(56) Entgegenhaltungen:
- EP-A1- 1 642 609
- EP-A1- 1 982 747
- WO-A1-93/15792
- WO-A1-2009/118066
- WO-A1-2010/076706
- WO-A1-2013/124615
- WO-A2-2014/020527

## Beschreibung

Die Erfindung betrifft eine tragbare Vorrichtung für die optische Signalübermittlung vom und zum menschlichen Auge gemäß dem Oberbegriff von Anspruch 1.

Eine solche Vorrichtung soll insbesondere der Lichttherapie dienen. Zahlreiche Befindlichkeitsstörungen des Menschen sind auf Lichtmangel insbesondere im blauen Frequenzbereich zurückzuführen. Eine dieser Befindlichkeitsstörungen ist als "Winterdepression" bekannt. Im Allgemeinen tritt die Winterdepression oder saisonalaffektive Störung (SAD - Seasonal Affective Disorder), bei welcher es sich um eine depressive Störung handelt, in den Herbst- und Wintermonaten auf, wobei neben den depressiven Symptomen, beispielsweise in Form einer bedrückten Stimmung, Reduzierung des Energieniveaus und Ängstlichkeit, noch atypische Symptome wie Verlängerung der Schlafdauer, verstärkter Appetit auf Süßigkeiten, Gewichtszunahme, erhöhte Selbstmordrate, erhöhte Unfallhäufigkeit usw. hinzukommen. Diese Beschwerden sind darauf zurückzuführen, dass im Normalfall spezielle Ganglienzellen in der Netzhaut auf blaues Licht reagieren und die Ausschüttung von Melatonin unterdrücken. Fehlt dieser blaue Lichtanteil durch wenig Himmelslicht im Winter, können verschiedene Krankheitssymptome wie eben das SAD-Syndrom auftreten.

Diese Symptome können durch künstliche Zufuhr blauen Lichtes bekämpft werden. Blaulicht ist erwiesenermaßen therapeutisch wirksam und wirkt nicht nur bei saisonaler Depression, sondern auch generell bei Energie- und Lichtmangel, Jetlag, oder beim prämenstruellen Syndrom (PMS).

Daher wurden bereits verschiedene Vorrichtungen vorgeschlagen, um blaues Licht künstlich zu erzeugen und dem menschlichen Auge zuzuführen. Zumeist sind diese Vorrichtungen als stationäre Geräte ausgeführt, vor denen die zu behandelnde Person zu sitzen oder zu liegen hat. Diese Geräte verfügen somit über den Nachteil, dass eine mit ihnen durchgeführte Therapie schwer in den Alltag zu integrieren ist. Es wären stattdessen Geräte zu bevorzugen, die keine Einschränkung der Alltagsaktivitäten erfordern. Hierfür wurden in der WO 2009/118066 A1, WO 93/15792 A1, WO 2013/124615 A1, EP 1 642 609 A1 und der WO 2010/076706 A1 Vorrichtungen vorgeschlagen, die am Kopf getragen werden können. Weitere Vorrichtungen wurden in der EP 1982747 A1 und der WO 2014/020527 A2 beschrieben.

Es ist daher Aufgabe der Erfindung eine tragbare Vorrichtung für die optische Signalübermittlung vom und zum menschlichen Auge bereit zu stellen, die mit hohem Tragekomfort unauffällig in Kopfnähe getragen werden kann und insbesondere ein zeitgleiches Tragen der Vorrichtung mit handelsüblichen Brillen ermöglicht. Des Weiteren sollen die Handhabung, die Aufbewahrung und der Transport entsprechender Vorrichtungen erleichtert werden.

Diese Aufgaben werden durch die Merkmale von Anspruch 1 gelöst. Anspruch 1 bezieht sich auf eine tragbare Vorrichtung für die optische Signalübermittlung vom und zum menschlichen Auge mit zumindest einem Emitter und/oder Detektor elektromagnetischer Wellen, einer Stromversorgung für den jeweils zumindest einen Emitter und/oder Detektor sowie eine Steuereinheit, die mit dem zumindest einen Emitter und/oder Detektor verbunden ist, bei der erfingdungsgemäß vorgeschlagen wird, dass jeweils zumindest ein Emitter und/oder Detektor elektromagnetischer Wellen an den beiden Endbereichen eines stabförmigen Gehäuses angeordnet sind, das in seinem mittleren Bereich eine Halteklemme aufweist, und die Stromversorgung für den jeweils zumindest einen Emitter und/oder Detektor sowie die Steuereinheit, die mit dem zumindest einen Emitter und/oder Detektor verbunden ist, innerhalb des stabförmigen Gehäuses angeordnet sind.

Die erfindungsgemäße Vorrichtung wird in Gebrauchslage im Nasenwurzelbereich des Anwenders platziert, wobei es mithilfe der Halteklemme entweder unmittelbar auf die Nase festgeklemmt werden kann, oder am Mittensteg einer Brille. Aufgrund der kleinen Abmessungen und des geringen Gewichts der erfindungsgemäßen Vorrichtung treten bei Kopfbewegungen nur kleine Kräfte auf, sodass die Vorrichtung durch die Halteklemme ausreichend sicher am Kopf gehalten wird. Die Halteklemme kann zur Verbesserung des Klemmsitzes auch eine gummiartige Beschichtung oder dergleichen aufweisen. Des Weiteren können die Klemmschenkel der Halteklemme zur Anpassung an die jeweilige Nasenform auch in ihrem Abstand zueinander einstellbar sein. An den beiden Endbereichen des stabförmigen Gehäuses ist jeweils zumindest ein Emitter und/oder Detektor elektromagnetischer Wellen angeordnet, der in Gebrauchslage jeweils in Richtung der Augen des Anwenders gerichtet ist. Bei dem genannten Emitter handelt es sich insbesondere um Lichtquellen zur Emission von Licht im sichtbaren Spektralbereich, die das generierte Licht unmittelbar in das jeweilige Auge des Anwenders abstrahlen. In einer kostengünstigen Variante können etwa blaue LEDs eingesetzt werden, die sich für lichttherapeutische Zwecke eignen und bereits mit einer entsprechenden Bündelungslinse ausgestattet sind. In einer aufwändigeren Variante können aber auch RGB-LEDs verwendet werden, die ebenfalls das therapeutische Blaulicht emittieren können, zusätzlich aber auch rote und grüne Spektralbereiche aufweisen und somit gemeinsam mit dem Blaulicht beliebige Lichtfarben generieren können. Als Emitter wird im Folgenden in herkömmlicher Weise ein aktiver Emitter verstanden, also ein Emitter, der elektromagnetische Wellen generiert und emittiert anstatt sie bloß zu reflektieren. Dabei kann die Generierung der elektromagnetischen Wellen und deren Emission aus dem stabförmigen Gehäuse auch örtlich getrennt voneinander stattfinden, etwa mithilfe eines innerhalb des stabförmigen Gehäuses angeordneten Lichtwellengenerators, der optisch mit den in den Endbereichen angeordneten Emitter, die das innerhalb des stabförmigen Gehäuses generierte Licht aus dem stabförmigen Gehäuse auskoppeln, verbunden ist. Allerdings wird etwa auch eine Anzeige, etwa eine LED-Anzeige, im Folgenden als Emitter aufgefasst, die etwa zu Zwecken des Biofeedback eingesetzt wird. Bei dem Detektor kann es sich etwa um einen Helligkeitssensor oder eine Kamera handeln, um die Helligkeit des Umgebungslichtes messen und je nach gemessener Helligkeit die Stärke des vom Emitter abgestrahlten Lichtes steuern zu können. Bei dem Detektor kann es sich aber auch etwa um einen Sensor für die Augenbewegungen oder Pupillenveränderungen des Anwenders handeln, um hieraus Rückschlüsse für eine therapeutisch optimierte Ansteuerung der Emitter zu gewinnen.

Der einfache Aufbau der erfindungsgemäßen Vorrichtung eignet sich insbesondere zur individuellen Anpassung an den jeweiligen Anwender. Vorzugsweise ist hierfür vorgesehen, dass das stabförmige Gehäuse relativ zur Halteklemme um seine Längsachse drehbar ist. Auf diese Weise kann der Winkel der optischen Achse des Emitters und/oder Detektors zu einer Horizontalebene verstellt werden. Zusätzlich kann vorgesehen sein, dass der jeweils zumindest eine Emitter und/oder Detektor relativ zum stabförmigen Gehäuse um eine zur Längsachse des stabförmigen Gehäuses senkrechte Drehachse dreh- oder schwenkbar ist. Auf diese Weise kann der Winkel der optischen Achse des Emitters und/oder Detektors zu einer Vertikalebene verstellt werden. Des Weiteren kann vorgesehen sein, dass das stabförmige Gehäuse längenverstellbar ist, um es auf unterschiedliche Augenabstände einstellen zu können.

Gemäß einer bevorzugten Ausführungsform handelt es sich bei dem zumindest einen Emitter um eine Lichtquelle oder Lichtquellengruppe. Die Verwendung einer Lichtquellengruppe verfügt über mehrere Vorteile. So kann etwa die Lichtstärke der einzelnen Lichtquellen verringert und die Gesamtlichtstärke über eine größere Fläche verteilt werden, um eine blendende Wirkung somit zu vermeiden. Andererseits kann aber auch durch Ansteuerung einzelner Lichtquellen eine Anpassung an unterschiedliche Augenabstände vorgenommen werden, ohne eine Längenverstellbarkeit des stabförmigen Gehäuses zu benötigen. Insbesondere für eine solche Anwendung ist es vorteilhaft, innerhalb des stabförmigen Gehäuses eine Steuereinheit anzuordnen, die mit dem zumindest einen Emitter und/oder Detektor verbunden ist, wobei die Lichtquellen einer Lichtquellengruppe von der Steuereinheit unabhängig voneinander ansteuerbar sind. Mithilfe einer solchen Steuereinheit ist es allerdings auch möglich, die Lichtquellen nach dem Einschalten nicht sofort auf eine maximale Leuchtstärke einzustellen, sondern eine einem Sonnenaufgang ähnliche Helligkeitssteigerung begleitend von einer entsprechenden Farbänderung von orangerot über gelb bis weiß und blau vorzunehmen. Ähnliches gilt für die Ausschaltphase, bei der ein Sonnenuntergang simuliert werden kann.

Für aufwändigere Steuerprogramme dieser Art ist es vorteilhaft, wenn das stabförmige Gehäuse über eine drahtlose Verbindung mit einem externen Gerät verbindbar ist. Auf diese Weise kann die Steuereinheit mit einem externen Gerät, vorzugsweise einem Mobiltelefon, mittels einer Drahtlosverbindung verbunden werden, um Rechenleistung auf das externe Gerät auszulagern und eine einfache graphische Benutzerführung etwa zum Auswählen von Farbvarianten, Lichtemissionsabläufen oder einer benutzerdefinierten, zeitgesteuerten Lichtemission zu ermöglichen. Auf dem externen Gerät kann auch der Status der erfindungsgemäßen Vorrichtung angezeigt werden wie z.B. der Ladezustand der Stromversorgung. Des Weiteren kann die erfindungsgemäße Vorrichtung auf diese Weise auch zum Gegenstand entsprechend spezialisierter Anwendungssoftware von Mobilgeräten ("Apps") werden.

Für die Optimierung der Signalübermittlung zwischen dem Emitter und/oder dem Detektor und dem Auge des Anwenders steht eine Vielzahl optischer Einrichtungen zur Verfügung. So können etwa Fresnellinsen in den Endbereichen des stabförmigen Gehäuses angeordnet werden, um das Emissionsprofil von Lichtquellen zu formen, wobei der Emitter relativ zur ihr zugeordneten Fresnellinse auch verschiebbar sein kann, um zusätzliche Emissionsprofile zu generieren. In einer bevorzugten Ausführungsform kann das stabförmige Gehäuse mit einer Aufsteckhülse versehen sein, die optische Elemente zur Formung von Abstrahlwinkel und/oder Helligkeit des Emitters aufweist.

Die Erfindung wird in weiterer Folge anhand von Ausführungsbeispielen mithilfe der beiliegenden Figuren näher erläutert. Es zeigen hierbei die
Fig. 1 eine perspektivische Ansicht einer Ausführungsform der erfindungsgemäßen Vorrichtung in Gebrauchslage,
Fig. 2 eine Ansicht einer Ausführungsform der erfindungsgemäßen Vorrichtung vom Anwender aus gesehen,
Fig. 3 eine Ansicht einer Ausführungsform der erfindungsgemäßen Vorrichtung von oben gesehen mit durch Pfeile angedeutete Hauptstrahlrichtung der Emitter,
Fig. 4a und 4b Ansichten einer Ausführungsform der erfindungsgemäßen Vorrichtung mit jeweils unterschiedlichem Abstand der Klemmschenkel der Halteklemme vom Anwender aus gesehen,
Fig. 5a und 5b seitliche Ansichten einer Ausführungsform der erfindungsgemäßen Vorrichtung bei unterschiedlicher Drehstellung des stabförmigen Gehäuses relativ zur Halteklemme,
Fig. 6a eine Ansicht einer weiteren Ausführungsform der erfindungsgemäßen Vorrichtung zur Befestigung an einer Brille vom Anwender aus gesehen,
Fig. 6b eine seitliche Ansicht der an einer Brille befestigten Ausführungsform gemäß der Fig. 6a,
Fig. 7 eine perspektivische Ansicht der an einer Brille befestigten Ausführungsform gemäß der Fig. 6,
Fig. 8 eine Schnittansicht einer Ausführungsform der erfindungsgemäßen Vorrichtung mit Emitter, Stromversorgung, Steuereinheit und Ladekontakten,
Fig. 9a und 9b Detailansichten zweier Abstrahlstellungen eines endseitig angeordneten Emitters auf einer biegsamen Platine,
Fig. 10a und 10b Detailansichten zweier Abstrahlstellungen eines endseitig angeordneten Emitters, der relativ zu einer Fresnellinse verschiebbar angeordnet ist,
Fig. 11a-11c Detailansichten unterschiedlicher Abstrahlwinkel eines in Form einer Lichtquellengruppe ausgeführten, endseitig angeordneten Emitters durch Ansteuerung unterschiedlicher Lichtquellen der Lichtquellengruppe,
Fig. 12a und 12b unterschiedliche Abstrahlwinkel eines endseitig angeordneten Emitters aufgrund unterschiedlicher Drehstellungen einer hülsenförmig ausgeführten Fresnellinse senkrecht zur Längsachse des stabförmigen Gehäuses gesehen,
Fig. 12c die Ausführungsform gemäß der Fig. 12a und 12b in Längsrichtung des stabförmigen Gehäuses gesehen,
Fig. 13 eine Ansicht einer weiteren Ausführungsform der erfindungsgemäßen Vorrichtung mit relativ zum stabförmigen Gehäuse schwenkbar ausgeführten Emitter von oben gesehen,
Fig. 14 eine Ansicht einer weiteren Ausführungsform der erfindungsgemäßen Vorrichtung mit zwei jeweils als Lichtquellengruppen ausgeführten Emitter vom Anwender aus gesehen,
Fig. 15 eine weitere Ausführungsform der erfindungsgemäßen Vorrichtung mit zusätzlichen Kameras und Helligkeitssensoren von oben gesehen,
Fig. 16 eine weitere Ausführungsform der erfindungsgemäßen Vorrichtung in Datenverbindung mit einem externen Gerät,
Fig. 17 eine weitere Ausführungsform der erfindungsgemäßen Vorrichtung mit zwei beidseitig angeordneten Filterscheiben vom Anwender aus gesehen,
Fig. 18 eine mögliche Lichtfarbkurve zur Simulation eines Sonnenaufganges und Sonnenunterganges,
Fig. 19 eine weitere Ausführungsform der erfindungsgemäßen Vorrichtung mit einer optischen Aufsteckhülse vom Anwender aus gesehen,
Fig. 20a und 20b eine weitere Ausführungsform der erfindungsgemäßen Vorrichtung mit längenverstellbarem Gehäuse bei zwei unterschiedlichen Längenstellungen vom Anwender aus gesehen,
Fig. 21 eine weitere Ausführungsform der erfindungsgemäßen Vorrichtung mit dünnen Seitenträgern vom Anwender aus gesehen,
Fig. 22a eine weitere Ausführungsform der erfindungsgemäßen Vorrichtung mit aufsteckbaren Hüllen im demontierten Zustand,
Fig. 22b die Ausführungsform gemäß der Fig. 22a im montierten Zustand,
Fig. 23a eine weitere Ausführungsform der erfindungsgemäßen Vorrichtung mit aufsteckbaren Hüllen im demontierten Zustand,
Fig. 23b die Ausführungsform gemäß der Fig. 23a im montierten Zustand,
Fig. 24 eine Ausführungsform eines Aufbewahrungs- und Ladebehälters mit Aufladekontakten, Magnet und Stromquellen,
Fig. 25 der Aufbewahrungs- und Ladebehälter gemäß der Fig. 24 mit eingelegter Vorrichtung gemäß der Erfindung,
Fig. 26 der Aufbewahrungs- und Ladebehälter gemäß der Fig. 24 und 25 mit eingelegter Vorrichtung gemäß der Erfindung im geöffneten Zustand,
Fig. 27 eine weitere Ausführungsform eines Aufbewahrungs- und Ladebehälters für die erfindungsgemäße Vorrichtung, und die
Fig. 28 der Aufbewahrungs- und Ladebehälter gemäß der Fig. 27 im geöffneten Zustand.

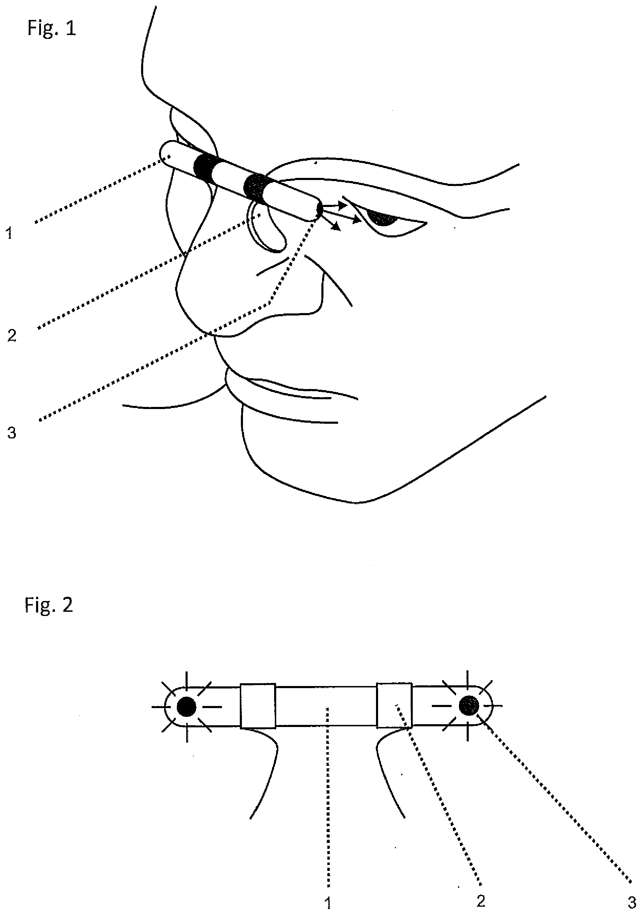

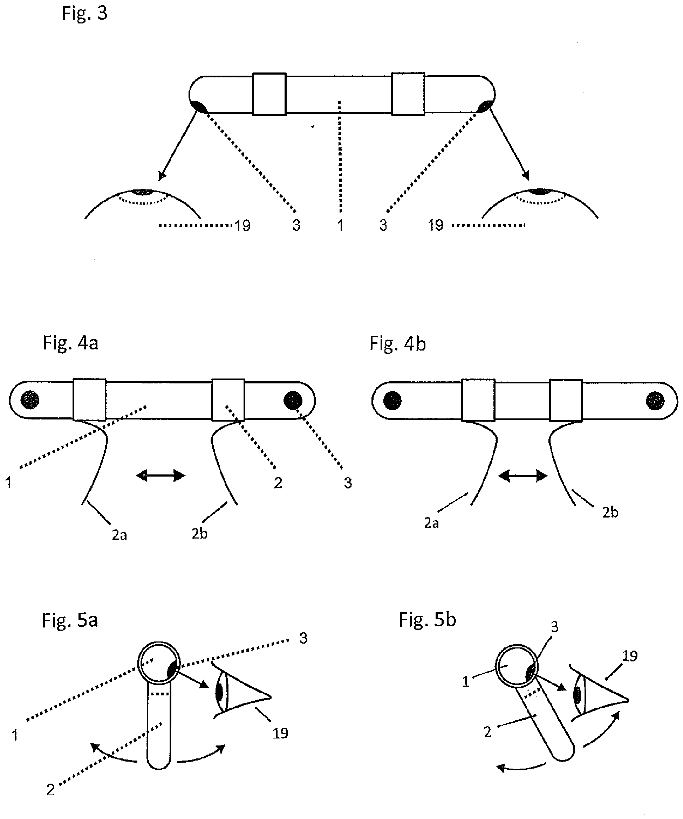

Zunächst zeigt die Fig. 1 eine perspektivische Ansicht einer Ausführungsform der erfindungsgemäßen Vorrichtung in Gebrauchslage, wobei das stabförmige Gehäuse 1 mithilfe der Halteklemme 2 an der Nase des Anwenders befestigt ist. Die Halteklemme 2 ist dabei in Form zweier vorzugsweise federnder Klemmschenkel 2a, 2b ausgeführt. An den beiden Endbereichen des stabförmigen Gehäuses 1 ist jeweils ein Emitter 3 und/oder Detektor angeordnet. Im gezeigten Ausführungsbeispiel handelt es sich etwa um jeweils eine Lichtquelle, die sichtbares Licht in die Augen des Anwenders abstrahlen. Die Fig. 2 zeigt eine Ansicht der Ausführungsform der erfindungsgemäßen Vorrichtung gemäß Fig. 1 vom Anwender aus gesehen und die Fig. 3 eine Ansicht dieser Ausführungsform der erfindungsgemäßen Vorrichtung von oben gesehen mit durch Pfeile angedeutete Hauptstrahlrichtung der Emitter 3. Dabei ist ersichtlich, dass jedem der beiden Emitter 3 ein Auge des Anwenders zugeordnet ist und die Hauptstrahlrichtung des Emitters 3 so auszurichten ist, dass eine bestmögliche Abstrahlung des Lichts in das ihm jeweils zugeordnete Auge 19 des Anwenders erfolgt. Die Hauptstrahlrichtungen sind dabei in der Fig. 3 mit Pfeilen angedeutet.

Um den Klemmsitz der Halteklemme 2 an der Nase des Anwenders zu optimieren kann vorgesehen sein, dass die beiden Klemmschenkel 2a, 2b der Halteklemme 2 in ihrem Abstand zueinander verstellbar sind, wie anhand der Fig. 4 gezeigt wird. Die Fig. 4a und 4b zeigen dabei Ansichten mit jeweils unterschiedlichem Abstand der Klemmschenkel 2a, 2b der Halteklemme 2 vom Anwender aus gesehen.

Um den Winkel der optischen Achse der Emitter 3 relativ zu einer Horizontalebene verstellen zu können, kann das stabförmige Gehäuse 1 relativ zur Halteklemme 2 um die Längsachse des stabförmigen Gehäuses 1 verdrehbar ausgeführt sein wie anhand der Fig. 5 gezeigt wird. Die Fig. 5a und 5b zeigen dabei seitliche Ansichten dieser Ausführungsform der erfindungsgemäßen Vorrichtung bei unterschiedlicher Drehstellung des stabförmigen Gehäuses 1 relativ zur Halteklemme 2, wobei die geradlinigen Pfeile die Hauptstrahlrichtung eines Emitters 3 andeuten, und die gekrümmten Peile die Drehrichtungen der Halteklemme 2 relativ zum stabförmigen Gehäuse 1.

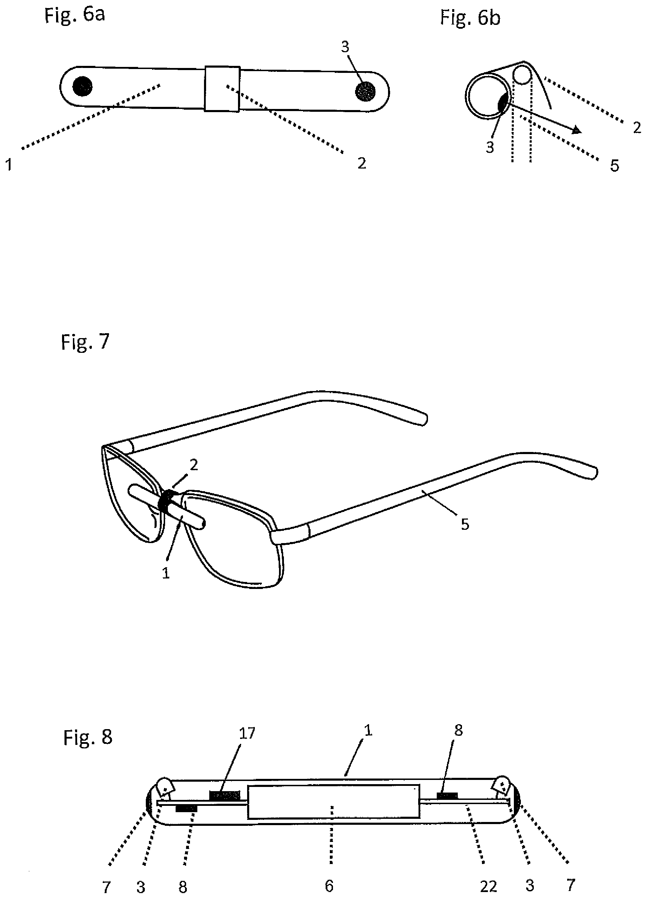

Die erfindungsgemäße Vorrichtung kann auch an einer Brille 5 befestigt werden, wie anhand der Fig. 6 und 7 gezeigt wird. Hierzu kann die Halteklemme 2 entsprechend ausgeführt sein, etwa in Form eines Haltebügels, wie in der Fig. 6b ersichtlich ist. Da für die sichere Befestigung an der Brille 5 jedenfalls eine Mindestklemmkraft erforderlich ist, wird im Folgenden auch eine zu einem Haltebügel ähnliche Ausführung als Halteklemme 2 verstanden. Die Fig. 6a zeigt dabei eine Ansicht einer solchen Ausführungsform vom Anwender aus gesehen und die Fig. 6b eine seitliche Ansicht. In der Fig. 7 ist eine perspektivische Ansicht der an einer Brille 5 befestigten Ausführungsform gemäß der Fig. 6 ersichtlich.

Anhand der Fig. 8 wird ein möglicher Aufbau einer erfindungsgemäßen Vorrichtung erläutert. Innerhalb des stabförmigen Gehäuses 1 ist eine Stromversorgung 6 angeordnet, die leitend mit Ladekontakten 7 verbunden ist, die sich jeweils an den beiden Enden des stabförmigen Gehäuses 1 befinden. Des Weiteren ist eine Steuereinheit 8 für den Emitter 3 und/oder Detektor innerhalb des stabförmigen Gehäuses 1 vorgesehen, wobei im gezeigten Ausführungsbeispiel jeder der beiden Emitter 3 mit einer eigenen Steuereinheit 8 angesteuert wird. In den beiden Endbereichen des stabförmigen Gehäuses 1 ist jeweils ein Emitter 3 angeordnet, im gezeigten Ausführungsbeispiel jeweils eine LED-Leuchte. Ferner kann innerhalb des stabförmigen Gehäuses 1 noch ein Magnetsensor 17 vorgesehen sein, dessen Funktion in weiterer Folge noch erläutert werden wird.

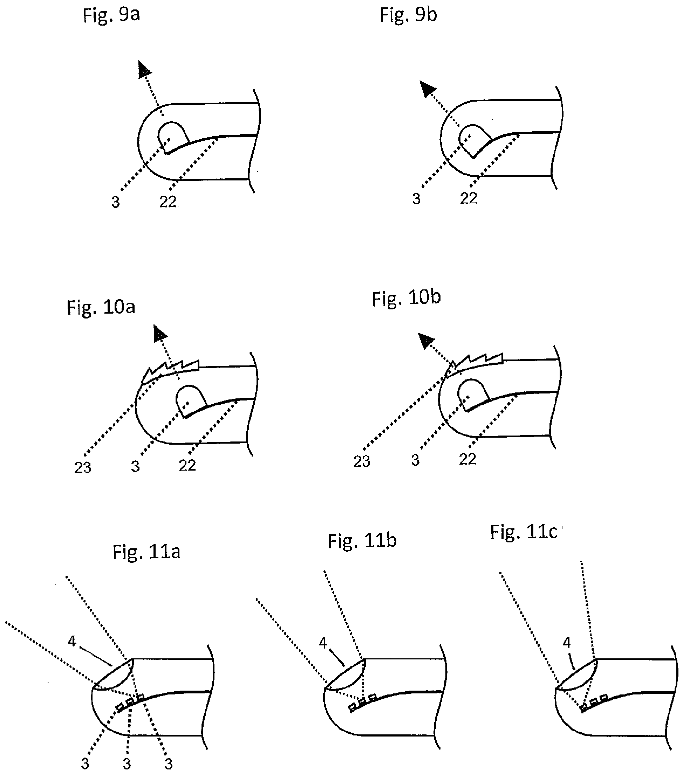

Der Emitter 3 und/oder Detektor können jeweils über eine Platine 22 mit der Stromversorgung 6 und der ihnen jeweils zugeordneten Steuereinheit 8 verbunden sein, die etwa auch biegsam ausgeführt sein kann. Die biegsame Ausführung verfügt etwa auch über den Vorteil, dass der Abstrahlwinkel der Emitter 3 verändert werden kann, wie anhand der Fig. 9 erläutert wird. Die Fig. 9a und 9b zeigen dabei Detailansichten zweier Abstrahlstellungen eines endseitig angeordneten Emitters 3 auf einer biegsamen Platine 22, wobei je nach Biegestellung der Platine 22 der Winkel der Hauptstrahlrichtung des betreffenden Emitters 3 relativ zu einer Vertikalebene verändert werden kann.

Freilich kann die Hauptstrahlrichtung der Emitter 3 auch durch optische Einrichtungen verändert werden. Die Fig. 10 zeigt hierzu ein Beispiel mithilfe einer Fresnellinse 23, die jeweils in den beiden Endbereichen des stabförmigen Gehäuses 1 im Abstrahlbereich des betreffenden Emitters 3 angeordnet ist. Die Fig. 10a und 10b zeigen hierbei Detailansichten zweier Abstrahlstellungen eines endseitig angeordneten Emitters 3, der relativ zur ihm zugeordneten Fresnellinse 23 verschiebbar angeordnet ist. Die Lage des Emitters 3 kann dabei werkseitig eingestellt werden, oder mithilfe von Verschiebemechaniken von außen vorgenommen werden.

Eine weitere Möglichkeit zur Veränderung der Hauptstrahlrichtungen der Emitter 3 ist in der Fig. 11 dargestellt. Die Fig. 11a-11c zeigen dabei Detailansichten unterschiedlicher Abstrahlwinkel dreier in Form einer Lichtquellengruppe ausgeführter, endseitig angeordneter Emitter 3 durch Ansteuerung unterschiedlicher Emitter 3 der Lichtquellengruppe. Die geeignete Ansteuerung der Lichtquellengruppe erfolgt dabei über die Steuereinheit 8. Die unterschiedliche Hauptstrahlrichtung ergibt sich dabei durch eine unterschiedliche Positionierung des ausgewählten Emitters 3 der Lichtquellengruppe relativ zu einer Abstrahlöffnung 4 im stabförmigen Gehäuse 1, die im Abstrahlbereich der Emitter 3 angeordnet ist.

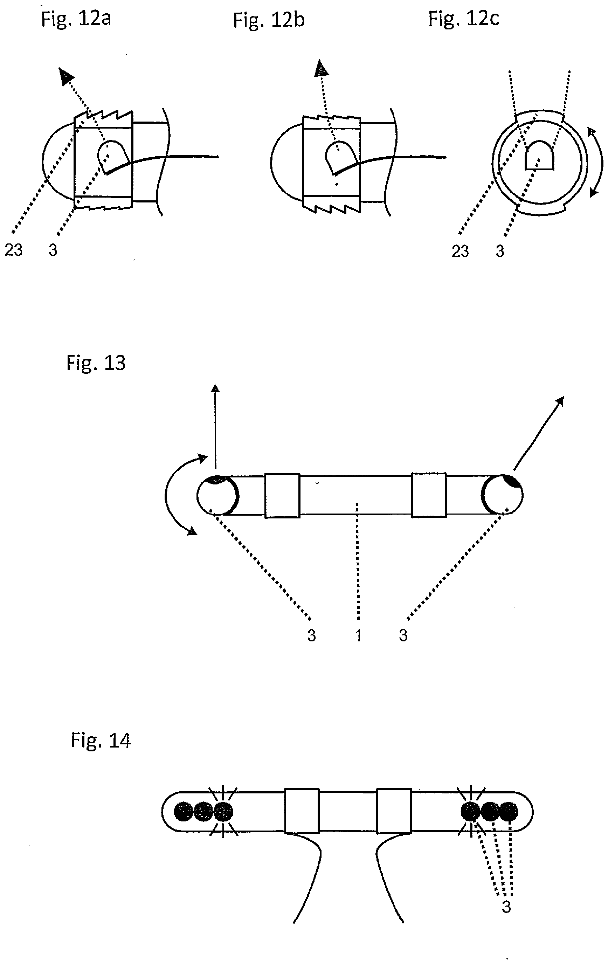

Die Fig. 12 zeigt eine zur Fig. 10 vergleichbare Ausführung, wobei unterschiedliche Abstrahlwinkel eines endseitig angeordneten Emitters 3 aufgrund unterschiedlicher Drehstellungen einer hülsenförmig ausgeführten Fresnellinse 23 bewerkstelligt werden, wobei die hülsenförmig ausgeführte Fresnellinse 23 um die Längsachse des stabförmigen Gehäuses 1 verdrehbar ist. Die Verdrehbarkeit ist deutlich in der Fig. 12c ersichtlich, die diese Ausführungsform gemäß der Fig. 12a und 12b in Längsrichtung des stabförmigen Gehäuses 1 gesehen zeigt. Die gekrümmten Pfeile deuten dabei die Drehrichtungen der hülsenförmig ausgeführten Fresnellinse 23 an, die in ihrem Umfangsbereich mit unterschiedlichen optischen Eigenschaften gefertigt ist. Je nachdem welcher Umfangsabschnitt sich in der Abstrahlöffnung 4 des betreffenden Emitters 3 befindet, wird die Hauptstrahlrichtung des abgestrahlten Lichtes unterschiedlich orientiert sein, wie anhand der gekrümmten Pfeile in den Fig. 12a und 12b angedeutet ist.

Eine weitere Möglichkeit zur Veränderung der Hauptstrahlrichtungen der Emitter 3 ist in der Fig. 13 gezeigt. Hierbei sind die endseitig angeordneten Emitter 3 jeweils relativ zum stabförmigen Gehäuse 1 schwenkbar ausgeführt, wobei die Schwenkachse senkrecht zur Längsachse des stabförmigen Gehäuses 1 orientiert ist. Die Hauptstrahlrichtungen der Emitter 3 sind dabei durch geradlinige Pfeile angedeutet und die Schwenkrichtung durch gekrümmte Pfeile.

In der Fig. 14 ist eine Ansicht einer weiteren Ausführungsform der erfindungsgemäßen Vorrichtung mit zwei jeweils als Lichtquellengruppen ausgeführten Emitter 3 dargestellt. Mithilfe einer solchen Ausführungsform kann einerseits eine Einstellung auf unterschiedliche Augenabstände vorgenommen werden, indem jeweils eine der Lichtquellen einer jeden Lichtquellengruppe aktiviert wird, oder andererseits die Lichtstärke eines jeden Emitters 3 reduziert werden, falls alle Emitter 3 einer Lichtquellengruppe aktiviert werden. Im letztgenannten Fall kann eine Blendwirkung auf den Anwender vermieden werden.

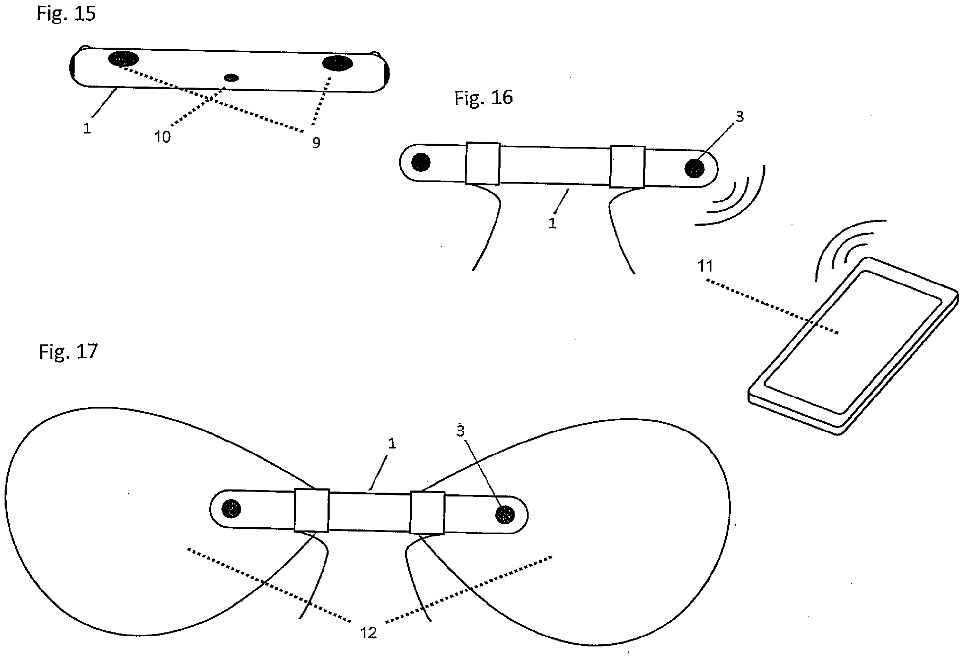

Die erfindungsgemäße Vorrichtung eignet sich auch als Träger zusätzlicher Komponenten, deren Anordnung in Augennähe vorteilhaft sein kann und die Tätigkeit der Emitter 3 und/oder Detektoren unterstützen. Die Fig. 15 zeigt etwa eine Ausführungsform der erfindungsgemäßen Vorrichtung mit zusätzlichen Kameras 9 und einem Helligkeitssensor 10. Die Kameras 9 können etwa dazu dienen Augenbewegungen oder Veränderungen der Pupillen des Anwenders zu detektieren. Der Helligkeitssensor 10 detektiert die Umgebungslichtstärke und reduziert gegebenenfalls die Lichtstärke der Emitter 3, um eine vorgegebene Gesamtlichtstärke auf die Augen des Anwenders nicht zu überschreiten.

Aufgrund der vielfältigen Bedienmöglichkeiten der erfindungsgemäßen Vorrichtung kann es zweckmäßig sein die Bedienung und Ansteuerung der Komponenten innerhalb des stabförmigen Gehäuses 1 auf ein externes Gerät 11 auszulagern, wie anhand der Fig. 16 ersichtlich ist. Bei dem externen Gerät 11 kann es sich etwa um ein Mobiltelefon handeln, das mittels einer Drahtlosverbindung mit einer Bedienschnittstelle des stabförmigen Gehäuses 1 verbunden werden kann.

Die Fig. 17 zeigt eine weitere Anwendungsmöglichkeit der erfindungsgemäßen Vorrichtung, bei der das stabförmige Gehäuse 1 mit zwei beidseitig ansteckbaren Filterscheiben 12 versehen wird. Bei diesen Filterscheiben 12 kann es sich etwa um farblich getönte Kunststofffolien handeln, die etwa eine orange Färbung aufweisen, um von außen kommendes, blaues Licht auszufiltern. Dadurch wird der gegenteilige Effekt des Blaulichtes erreicht, nämlich eine Aktivierung des Melatonins, wodurch nachteilige Wirkungen eines "Jetlag" vermieden werden können.

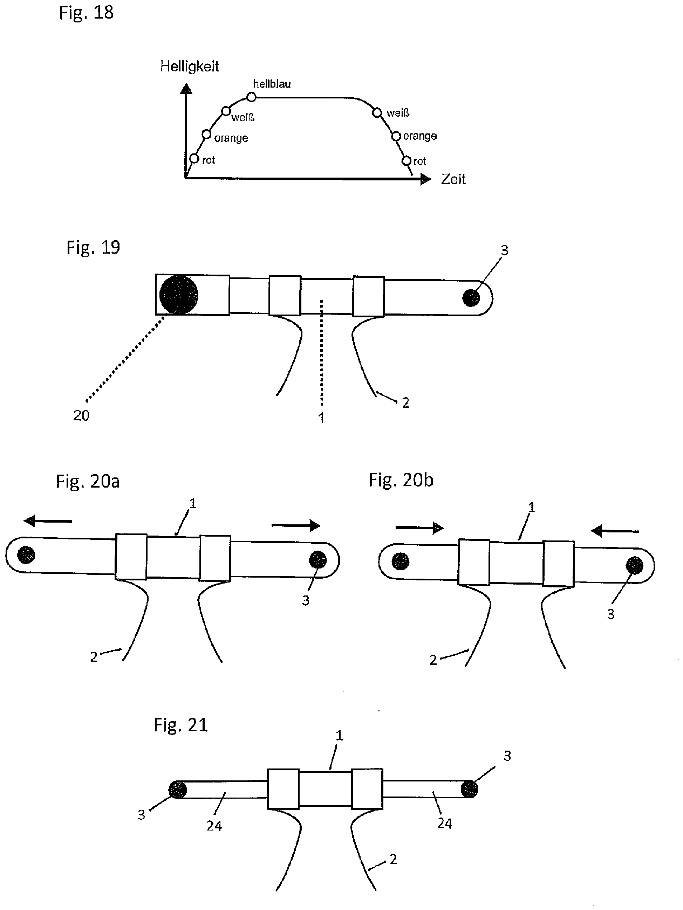

Die Fig. 18 zeigt einen möglichen Verlauf der Helligkeit des Lichts der Emitter 3 in Abhängigkeit der Zeit, wobei ersichtlich ist, dass nach Inbetriebnahme der Vorrichtung beispielsweise eine allmähliche Zunahme der Lichthelligkeit sowie ein Wechsel der Lichtfarbe von beginnendem Rot über Orange und Gelb bis zum letztendlichen Blaulicht bzw. Blau-Weißlicht stattfinden kann, um einen Sonnenaufgang zu simulieren. In ähnlicher Weise kann dieser Vorgang beim Ausschalten der Vorrichtung umgekehrt werden, um einen Sonnenuntergang zu simulieren. Die Simulierung eines Sonnenaufgangs bzw. Sonnenuntergangs kann etwa mit mehreren verschiedenfarbigen Emitter 3 (rot, grün und blau) bewerkstelligt werden.

Die Fig. 19 stellt eine weitere Ausführungsform der erfindungsgemäßen Vorrichtung mit einer optischen Aufsteckhülse 20 dar. Die Aufsteckhülse 20 wird in der gezeigten Ausführungsform in Richtung der Längsachse des stabförmigen Gehäuses 1 von außen aufgeschoben und bedeckt im aufgeschobenen Zustand die Abstrahlöffnung 4 des jeweiligen Emitters 3. Die Aufsteckhülse 20 ändert dabei die Abstrahlcharakteristik der Emitter 3, indem sie etwa als Diffusor wirkt oder den Raumwinkel des abgestrahlten Lichtes des betreffenden Emitters 3 aufweitet.

Eine weitere Möglichkeit zur Anpassung der erfindungsgemäßen Vorrichtung an unterschiedliche Augenabstände ist in der Fig. 20 gezeigt, indem das stabförmige Gehäuse 1 längenverstellbar ausgeführt ist. Die Fig. 20a und 20b zeigen dabei zwei unterschiedliche Längenstellungen des stabförmigen Gehäuses 1, die etwa durch einen Ausziehmechanismus in Richtung der dargestellten Pfeile verwirklicht werden können.

Eine besonders schlanke und elegante Ausführung ist in der Fig. 21 dargestellt, bei der die endseitig angeordneten Emitter 3 und/oder Detektoren an dünnen Seitenträgern 24 des stabförmigen Gehäuses 1 angeordnet sind. In diesem Fall weist das stabförmige Gehäuse 1 einen mittleren Bereich auf, an dem die Halteklemme 2 befestigt ist, sowie zwei Endbereiche, die in Form der beiden Seitenträger 24 ausgeführt sind. Diese Ausführungsform eignet sich besonders für eine Längenverstellbarkeit der gesamten Anordnung, indem die Seitenträger 24 des stabförmigen Gehäuses 1 relativ zum mittleren Bereich ein- und ausschiebbar sind.

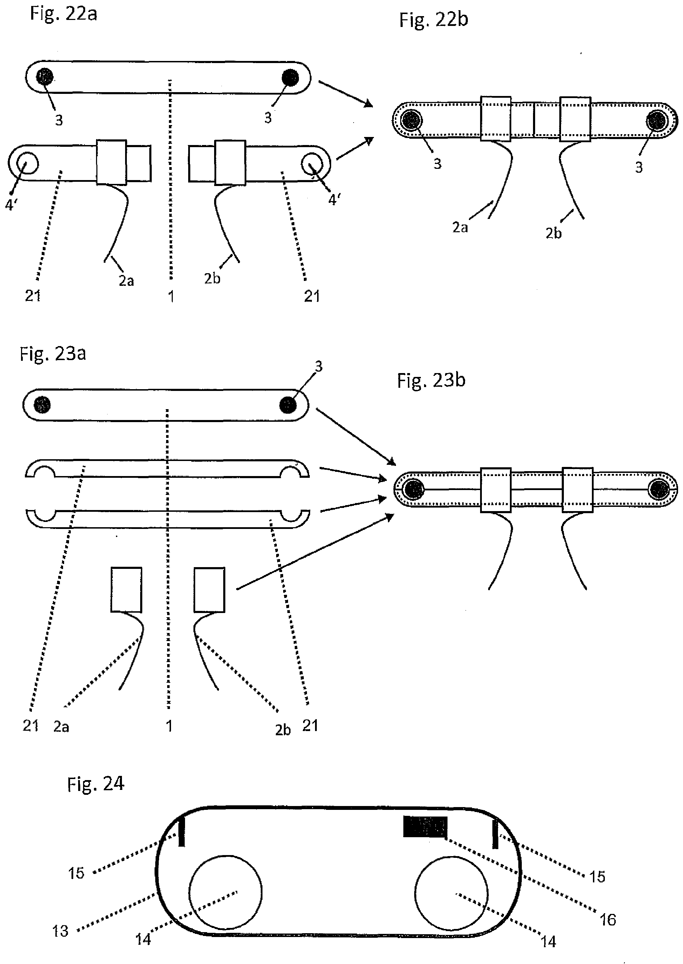

In der Fig. 22 ist eine weitere Ausführungsform der erfindungsgemäßen Vorrichtung dargestellt, bei der das stabförmige Gehäuse 1 mit beidseits auf seine Endbereiche in Längsrichtung des stabförmigen Gehäuses 1 aufsteckbaren Hüllen 21 versehen werden kann. Die Fig. 22a zeigt dabei das stabförmige Gehäuse 1 und die Hüllen 21 im demontierten Zustand, und die Fig. 22b die Ausführungsform gemäß der Fig. 22a im montierten Zustand. Die Hüllen 21 verfügen dabei jeweils über eine Abstrahlöffnung 4', die mit der Abstrahlöffnung 4 des stabförmigen Gehäuses 1 im montierten Zustand fluchtet. Die Hüllen 21 tragen des Weiteren jeweils eine der Klemmschenkel 2a, 2b, sodass sie im montierten Zustand die Halteklemme 2 bilden. Diese Ausführung verfügt über den Vorteil, dass die Hüllen 21 bei Abnutzung oder Beschädigung ausgewechselt werden können und das stabförmige Gehäuse 1 schützen.

Die Fig. 23a zeigt eine weitere Ausführungsform der erfindungsgemäßen Vorrichtung mit aufsteckbaren Hüllen 21 im demontierten Zustand, und die Fig. 23b die Ausführungsform gemäß der Fig. 23a im montierten Zustand. Dabei ist die Hülle 21 in Form zweier Klemmkörper aufgebaut, die das stabförmige Gehäuse 1 in dessen Querrichtung zwischen sich aufnehmen. In weiterer Folge können Befestigungshülsen für jeweils einen Klemmschenkel 2a, 2b auf die montierte Hülle 21 aufgeschoben werden, die im montierten Zustand wiederum die Halteklemme 2 bilden.

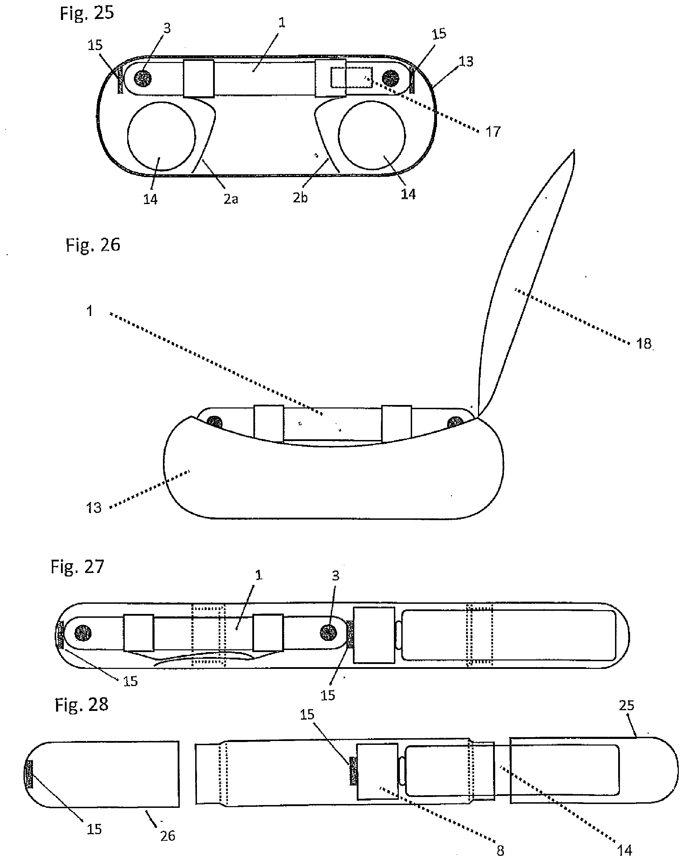

Die Fig. 24-26 zeigen eine Ausführungsform eines Aufbewahrungs- und Ladebehälters 13 für die erfindungsgemäße Vorrichtung. Der Aufbewahrungs- und Ladebehälter 13 ist mit Stromquellen 14 versehen, die mit Aufladekontakten 15 in leitender Verbindung stehen. Die Stromquellen 14 können etwa als Ladebatterien ausgeführt sein. Alternativ können die Stromquellen 14 auch als Akku ausgeführt sein, der über eine eigene Ladeelektronik etwa über einen Micro-USB-Anschluss aufladbar ist. Wie in der Fig. 25 ersichtlich ist befinden sich die Ladekontakte 7 des stabförmigen Gehäuses 1 (siehe Fig. 8) in einem elektrischen Kontakt zu den Aufladekontakten 15, sobald die erfindungsgemäße Vorrichtung in den Aufbewahrungs- und Ladebehälter 13 eingelegt wurde. Des Weiteren kann der Aufbewahrungs- und Ladebehälter 13 mit einem Magnet 16 versehen sein, der bei eingelegtem Gehäuse 1 nahe dem Magnetsensor 17, der innerhalb des stabförmigen Gehäuses 1 angeordnet ist, zu liegen kommt. Die innerhalb des stabförmigen Gehäuses 1 befindliche Steuereinheit 8 kann somit detektieren, ob die erfindungsgemäße Vorrichtung in den Aufbewahrungs- und Ladebehälter 13 eingelegt wurde und in weiterer Folge einen Ladeschaltkreis für die Stromversorgung 6 öffnen. Die erfindungsgemäße Vorrichtung kann dadurch aber auch erkennen, ob es dem Aufbewahrungs- und Ladebehälter 13 entnommen wurde und mit einem Leuchtzyklus beginnen oder ihn auch beenden, sobald es in den Aufbewahrungs- und Ladebehälter 13 eingelegt wurde. Die Fig. 26 zeigt den Aufbewahrungs- und Ladebehälter 13 gemäß der Fig. 24 und 25 mit eingelegter Vorrichtung gemäß der Erfindung bei geöffnetem Deckel 18 des Aufbewahrungs- und Ladebehälters 13. Der Aufbewahrungs- und Ladebehälter 13 kann sehr kompakt ausgeführt werden und kann daher jederzeit leicht und unauffällig vom Anwender mitgeführt werden.

Die Fig. 27 und 28 zeigen eine weitere Ausführungsform eines Aufbewahrungs- und Ladebehälters 13 für die erfindungsgemäße Vorrichtung, wobei die Fig. 27 den Aufbewahrungs- und Ladebehälter 13 mit eingelegter Vorrichtung gemäß der Erfindung und die Fig. 28 den Aufbewahrungs- und Ladebehälter 13 gemäß der Fig. 27 im geöffneten Zustand ohne der erfindungsgemäßen Vorrichtung darstellt. Diese Ausführungsform des Aufbewahrungs- und Ladebehälters 13 weist ebenfalls zwei Aufladekontakte 15 auf, wobei ein Aufladekontakt 15 in einer aufsteckbare Ladehülse 4 angeordnet ist, die auf einen mittleren Teil des Aufbewahrungs- und Ladebehälters 13 aufsteckbar ist. Ferner kann am gegenüberliegenden Ende des Aufbewahrungs- und Ladebehälters 13 eine weiterer Hülse 25 aufsteckbar sein, die etwa einen Micro-USB-Anschluss schützt. Des Weiteren ist eine Stromquelle 14 vorgesehen, die mit den Aufladekontakten 15 in leitender Verbindung steht. Falls die erfindungsgemäße Vorrichtung in den Aufbewahrungs- und Ladebehälter 13 eingelegt und die Ladehülse 4 aufgeschoben wurde, befinden sich die Ladekontakte 7 des stabförmigen Gehäuses 1 in einem elektrischen Kontakt zu den Aufladekontakten 15. Auch in diesem Fall kann der Aufbewahrungs- und Ladebehälter 13 mit einem Magnet 16 versehen sein (in der Fig. 27 und 28 nicht ersichtlich), der bei eingelegtem Gehäuse 1 nahe dem Magnetsensor 17, der innerhalb des stabförmigen Gehäuses 1 angeordnet ist, zu liegen kommt. Die innerhalb des stabförmigen Gehäuses 1 befindliche Steuereinheit 8 kann somit wiederum detektieren, ob die erfindungsgemäße Vorrichtung in den Aufbewahrungs- und Ladebehälter 13 eingelegt wurde und in weiterer Folge eine Ladeschaltkreis für die Stromversorgung 6 öffnen.

Die tragbare Vorrichtung gemäß der Erfindung kann somit sehr kompakt ausgeführt werden, wobei auch überaus praktische Aufbewahrungs- und Ladebehälter 13 vorgeschlagen werden. Die Erfindung stellt dabei eine tragbare Vorrichtung für die optische Signalübermittlung vom und zum menschlichen Auge bereit, die mit hohem Tragekomfort unauffällig in Kopfnähe getragen werden kann und insbesondere ein zeitgleiches Tragen der Vorrichtung mit handelsüblichen Brillen ermöglicht. Des Weiteren werden die Handhabung, die Aufbewahrung und der Transport entsprechender Vorrichtungen sehr erleichtert.

### Bezugszeichenliste:

- 1: stabförmiges Gehäuse
- 2: Halteklemme
- 3: Emitter
- 4: Abstrahlöffnung
- 5: Brille
- 6: Stromversorgung
- 7: Ladekontakte
- 8: Steuereinheit
- 9: Kamera
- 10: Helligkeitssensor
- 11: Externes Gerät
- 12: Ansteckbare Filterscheiben
- 13: Aufbewahrungs- und Ladebehälter
- 14: Stromquelle
- 15: Aufladekontakte
- 16: Magnet
- 17: Magnetsensor
- 18: Deckel
- 19: Auge
- 20: Optische Aufsteckhülse
- 21: Hülle
- 22: Platine
- 23: Fresnellinse
- 24: Seitenträger
- 25: Hülse
- 26: Ladehülse

## Patentansprüche

1. Tragbare Vorrichtung für die optische Signalübermittlung vom und zum menschlichen Auge mit zumindest einem Emitter (3) und/oder Detektor elektromagnetischer Wellen, einer Stromversorgung für den jeweils zumindest einen Emitter (3) und/oder Detektor sowie eine Steuereinheit (8), die mit dem zumindest einen Emitter (3) und/oder Detektor verbunden ist, **gekennzeichnet durch** ein stabförmiges Gehäuse (1), an dessen beiden Endbereichen jeweils zumindest ein Emitter (3) und/oder Detektor elektromagnetischer Wellen angeordnet sind, und das in seinem mittleren Bereich eine Halteklemme (2) aufweist, wobei die Stromversorgung für den jeweils zumindest einen Emitter (3) und/oder Detektor sowie die Steuereinheit (8), die mit dem zumindest einen Emitter (3) und/oder Detektor verbunden ist, innerhalb des stabförmigen Gehäuses (1) angeordnet sind.

2. Tragbare Vorrichtung nach Anspruch 1, **dadurch gekennzeichnet, dass** das stabförmige Gehäuse (1) relativ zur Halteklemme (2) um seine Längsachse drehbar ist.

3. Tragbare Vorrichtung nach Anspruch 1 oder 2, **dadurch gekennzeichnet, dass** der jeweils zumindest eine Emitter (3) und/oder Detektor relativ zum stabförmigen Gehäuse (1) um eine zur Längsachse des stabförmigen Gehäuses (1) senkrechte Drehachse dreh- oder schwenkbar ist.

4. Tragbare Vorrichtung nach einem der Ansprüche 1 bis 3, **dadurch gekennzeichnet, dass** das stabförmige Gehäuse (1) längenverstellbar ist.

5. Tragbare Vorrichtung nach einem der Ansprüche 1 bis 4, **dadurch gekennzeichnet, dass** es sich bei dem zumindest einen Emitter (3) um eine Lichtquelle oder Lichtquellengruppe handelt.

6. Tragbare Vorrichtung nach Anspruch 5, **dadurch gekennzeichnet, dass** die Lichtquellen einer Lichtquellengruppe von der Steuereinheit (8) unabhängig voneinander ansteuerbar sind.

7. Tragbare Vorrichtung nach einem der Ansprüche 1 bis 6, **dadurch gekennzeichnet, dass** das stabförmige Gehäuse (1) über eine drahtlose Verbindung mit einem externen Gerät (11) verbindbar ist.

8. Tragbare Vorrichtung nach einem der Ansprüche 1 bis 7, **dadurch gekennzeichnet, dass** das stabförmige Gehäuse (1) mit einer Aufsteckhülse (20) versehen ist, die optische Elemente zur Formung von Abstrahlwinkel und/oder Helligkeit des Emitters (3) aufweist.

## Claims

1. A portable device for optical signal transmission to and from the human eye, comprising at least one emitter (3) and/or detector of electromagnetic waves, a power supply for the respective at least one emitter (3) and/or detector and a control unit (8) which is connected to the at least one emitter (3) and/or detector, **characterized by** a rod-shaped housing (1), at the two end regions of which in each case at least one emitter (3) and/or detector of electromagnetic waves is arranged, and which comprises a holding clamp in its central region, wherein the power supply for the at least one emitter (3) and/or detector and the control unit (8), which is connected to the at least one emitter (3) and/or detector, is arranged within the rod-shaped housing (1).

2. Portable device according to claim 1, **characterized in that** the rod-shaped housing (1) is rotatable relative to the holding clamp (2) about its longitudinal axis.

3. Portable device according to claim 1 or 2, **characterized in that** the respective at least one emitter (3) and/or detector is rotatable or pivotable relative to rod-shaped housing (1) about an axis of rotation which is vertical to the longitudinal axis of the rod-shaped housing (1).

4. Portable device according to one of the claims 1 to 3, **characterized in that** the rod-shaped housing (1) is adjustable in length.

5. Portable device according to one of the claims 1 to 4, **characterized in that** the at least one emitter (3) is a light source or light source group.

6. Portable device according to claim 5, **characterized in that** the light sources of a light source group are independently controllable by the control unit (8).

7. Portable device according to one of the claims 1 to 6, **characterized in that** the rod-shaped housing (1) is connectable via a wireless connection to an external device (11).

8. Portable device according to one of the claims 1 to 7, **characterized in that** the rod-shaped housing (1) is provided with a plug-on sleeve (20) having optical elements for forming emission angle and/or brightness of the emitter (3).

## Revendications

1. Dispositif portatif pour la transmission optique de signaux en provenance et en direction de l'oeil humain avec au moins un émetteur (3) et/ou un détecteur d'ondes électromagnétiques, une alimentation électrique pour chaque au moins un émetteur (3) et/ou détecteur et une unité de commande (8) reliée à l'au moins un émetteur (3) et/ou détecteur, **caractérisé en ce qu'**il comporte un boîtier en forme de barre (1) dont les deux parties d'extrémité portent chacune au moins un émetteur (3) et/ou détecteur d'ondes électromagnétiques et qui présente dans sa partie médiane une pince de fixation (2), l'alimentation électrique de chaque au moins un émetteur (3) et/ou détecteur et l'unité de commande (8) qui est reliée à l'au moins un émetteur (3) et/ou détecteur étant disposées à l'intérieur du boîtier en forme de barre (1).

2. Dispositif portatif selon la revendication 1, **caractérisé en ce que** le boîtier en forme de barre (1) est capable de rotation autour de son axe longitudinal par rapport à la pince de fixation (2).

3. Dispositif portatif selon la revendication 1 ou 2, **caractérisé en ce que** chaque au moins un émetteur (3) et/ou détecteur est capable de rotation ou de pivotement par rapport au boîtier en forme de barre (1) autour d'un axe de rotation perpendiculaire à l'axe longitudinal du boîtier en forme de barre (1).

4. Dispositif portatif selon l'une des revendications 1 à 3, **caractérisé en ce que** le boîtier en forme de barre (1) est ajustable en longueur.

5. Dispositif portatif selon l'une des revendications 1 à 4, **caractérisé en ce que** l'au moins un émetteur (3) est une source lumineuse ou un groupe de sources lumineuses.

6. Dispositif portatif selon la revendication 5, **caractérisé en ce que** les sources lumineuses d'un groupe de sources lumineuses peuvent être commandées séparément les unes des autres par l'unité de commande (8).

7. Dispositif portatif selon l'une des revendications 1 à 6, **caractérisé en ce que** le boîtier en forme de barre (1) peut être relié par une liaison sans fil à un appareil externe (11).

8. Dispositif portatif selon l'une des revendications 1 à 7, **caractérisé en ce que** le boîtier en forme de barre (1) est muni d'une douille emboîtable (20) qui contient les éléments optiques servant à former l'angle de rayonnement et/ou la luminosité de l'émetteur (3).
